# DEMANDE DE BREVET EUROPEEN

(11) **EP 0 965 831 A1**
(43) Date de publication de la demande: **22.12.1999**
(21) Numéro de dépôt: 99400972.8
(22) Date de dépôt: 21.04.1999
(51) Int. Cl.: G01N 1/26

(54) **Installation d'analyse d'atmosphere**

(30) Priorité: 15.06.1998 FR 9807498
(71) Demandeur: L'AIR LIQUIDE, SOCIETE ANONYME POUR L'ETUDE ET L'EXPLOITATION DES PROCEDES GEORGES CLAUDE, 75321 Paris Cédex 07 (FR)
(72) Inventeur: Poynot, Philippe, 91190 Gif sur Yvette (FR)
(74) Mandataire: Mellul, Sylvie Lisette

(57) **Abrégé**

L'invention concerne une installation d'analyse en échantillonnage de la teneur en au moins un élément, d'au moins deux sources (S₁,S₂,..) de gaz initial, comportant des lignes de prélèvement (L_{Pi}), des lignes d'évacuation (L_{Ei}), des lignes secondaires (L_{Si}), des organes (Vᵢ) respectifs directeurs de flux, aptes à diriger chaque échantillon d'une source de gaz initiale considéré qui lui est associé vers sa ligne d'évacuation respective, ou, via sa ligne secondaire respective, vers un point de rassemblement avant analyse, une ligne tertiaire (L_{T}), reliée en sa partie amont au point de rassemblement et en sa partie aval à une évacuation (E), au moins une ligne d'analyse (L_{Ai})_{,} reliée en sa partie amont à la ligne tertiaire et en sa partie aval à un analyseur.

Une telle installation permet un prélèvement en continu, minimisant le temps de réponse de l'analyseur, et assurant au niveau de cet analyseur des échantillons de gaz représentatifs, elle permet par ailleurs de passer d'une source de gaz initiale à une autre, dans n'importe quel ordre, et avec une durée de consultation de chaque point de mesure parfaitement configurable

Figure 1 à reproduire.

## Description

La présente invention concerne le domaine des équipements d'analyse d'atmosphère et s'intéresse tout particulièrement au cas où il est nécessaire d'analyser un ou plusieurs éléments d'une atmosphère en plusieurs points d'une enceinte mettant en oeuvre cette atmosphère, ou d'un réseau de distribution de gaz, cela en mode multiplexage (mode discontinu : analyse tour à tour des points selon un ordre qui peut être variable et selon une durée de consultation de chaque point de mesure qui peut être également variable).

Ces applications d'analyse s'inscrivent dans un contexte où les utilisateurs de gaz industriels (traitement thermique, électronique, alimentaire etc..) ont de plus en plus couramment besoin d'analyser un ou plusieurs composants de l'atmosphère qu'ils mettent en oeuvre au niveau d'un poste utilisateur donné, ou encore une ou plusieurs impuretés (telles que oxygène ou encore vapeur d'eau) d'un gaz distribué avant son injection dans leur procédé, ceci afin d'être en mesure d'effectuer un contrôle qualité complet des pièces traitées, contrôle qualité complet supposant de pouvoir connaître dans quelles condition d'atmosphère chaque pièce a été traitée.

Les clients utilisateurs de gaz veulent donc couramment pouvoir connaître ces conditions d'atmosphère, voire les visualiser, les archiver, avoir une traçabilité, voire même traiter ces valeurs ainsi archivées.

On conçoit alors qu'il faut pouvoir être en mesure de proposer à ces clients, utilisateurs de gaz industriels, des méthodes et équipements d'analyse permettant de prélever des échantillons de gaz aux différents points d'analyse suivis (qu'il s'agisse de différents points d'une enceinte mettant en oeuvre l'atmosphère pour un traitement donné, ou de différents points d'analyse sur un réseau de distribution de gaz), permettant :
- de minimiser le temps de réponse du ou des analyseurs concernés ;
- d'assurer au niveau de la baie d'analyse l'approvisionnement en échantillons de gaz à analyser qui soient représentatifs de l'atmosphère de l'enceinte ou du gaz circulant dans le réseau de distribution à analyser, en particulier, on le conçoit, dans le cas où la baie d'analyse est éloignée des points de prélèvements ;
- d'effectuer les prélèvements et les analyses quel que soit l'ordre de chaque point de mesure ;
- d'effectuer les prélèvements et les analyses quelle que soit la durée de consultation d'un point de mesure donné, selon l'espèce analysée et le type d'analyseur mis en oeuvre.

On a proposé pour de telles applications de multiplexage l'utilisation de vannes rotatives basées sur l'avancement pas à pas de la vanne qui vient se placer tour à tour devant chaque canal à analyser. L'utilisation de telles vannes rotatives n'est cependant pas sans inconvénients, notamment liés à un matériel complexe, dont la robustesse à l'utilisation reste à démontrer, mais surtout dont un ordre d'avancement en rotation très précis doit être respecté.

La présente invention vise notamment à apporter des solutions aux problèmes techniques ci-dessus mentionnés.

L'installation d'analyse de la teneur en au moins un élément, d'au moins deux sources de gaz initiales, selon un mode d'échantillonnage, à l'aide d'au moins un analyseur apte à analyser ledit élément, selon l'invention, comporte alors :
- au moins deux sources de gaz initiales à analyser ;
- au moins deux lignes de prélèvement, chaque ligne étant reliée en sa partie amont à une desdites sources de gaz initiales, et en sa partie aval, à un organe respectif directeur de flux ;
- au moins deux lignes d'évacuation, chaque ligne d'évacuation étant reliée en sa partie amont à un desdits organes directeurs et en sa partie aval à une évacuation ou un point de stockage ;
- au moins deux lignes secondaires, chaque ligne secondaire étant reliée en sa partie amont à un desdits organes directeurs et, en sa partie aval, à un point de rassemblement, chaque organe directeur étant apte à diriger un échantillon de la source de gaz initiale qui lui est associée vers sa ligne d'évacuation respective ou vers le point de rassemblement via sa ligne secondaire respective ;
- une ligne tertiaire, reliée en sa partie amont au point de rassemblement et en sa partie aval à une évacuation ou un point de stockage ;
- au moins une ligne d'analyse, reliée en sa partie amont à la ligne tertiaire et en sa partie aval à au moins un desdits au moins un analyseur.

L'installation d'analyse selon l'invention pourra par ailleurs comporter l'une ou plusieurs des caractéristiques suivantes :
- un organe de pompage, situé entre le point de rassemblement et le point de connexion entre ligne d'analyse et ligne tertiaire ;
- la ligne tertiaire comporte, en aval du point de connexion de la ligne d'analyse sur la ligne tertiaire, un clapet anti-retour;
- la ligne tertiaire comporte, en aval du point de connexion de la ligne d'analyse sur la ligne tertiaire, un déverseur, et la ou au moins une des lignes d'analyse est munie d'un organe de création d'une perte de charge ;
- la ou au moins une des lignes d'évacuation est munie d'un organe de pompage ;
- la ou chaque ligne d'analyse est munie d'un organe directeur, permettant selon les cas, de diriger vers l'analyseur du gaz à analyser, un gaz de calibration, ou encore un gaz de purge ;
- le ou lesdits organes directeurs de l'installation sont constitués par une électrovanne trois voies ;
- le ou lesdits organes directeurs des lignes de prélèvement sont constitués par l'ensemble formé par le point de connexion des lignes de prélèvement, d'évacuation, et secondaire associées, chaque ligne secondaire et d'évacuation étant munie, en aval de ce point, d'un organe apte à permettre ou interrompre le passage de gaz par la ligne considérée ;
- les sources initiales à analyser sont à une pression supérieure à la pression atmosphérique ;
- les sources initiales à analyser sont à une pression sensiblement égale à la pression atmosphérique ou inférieure à la pression atmosphérique ;
- les sources initiales sont constituées par des échantillons de gaz pris sur un réseau de distribution ;
- les sources initiales sont constituées par des échantillons de gaz pris en différents points d'une enceinte mettant en oeuvre une atmosphère gazeuse ;
- l'installation comporte plusieurs analyseurs, les lignes d'analyse étant connectées sur la ligne tertiaire au niveau d'une nourrice ;
- l'installation comporte une seule ligne d'analyse connectant la ligne tertiaire et les analyseurs disposés en série ;
- l'installation comporte une ligne d'analyse par analyseur.

D'autres caractéristiques et avantages de la présente invention ressortiront de la description suivante de modes de réalisation donnée à titre illustratif mais nullement limitatif, faite en relation avec les dessins annexés, sur lesquels :
- la figure 1 est une représentation schématique d'une installation conforme à l'invention, mettant en oeuvre une seule ligne d'analyse et un seul analyseur ;
- la figure 2 est une représentation schématique d'une installation conforme à l'invention, comportant trois analyseurs et trois lignes d'analyse, connectées sur la ligne tertiaire au niveau d'une nourrice ;
- la figure 3 illustre un mode de réalisation d'un organe directeur selon l'invention, tel que constitué par l'ensemble formé par le point de connexion des lignes de prélèvement, d'évacuation, et secondaire associées, chaque ligne secondaire et d'évacuation étant munie d'une vanne deux voies ;
- les figures 4 et 5 illustrent deux variantes de mise en oeuvre de l'invention avec piquage de la ligne d'analyse sur la ligne tertiaire qui conduit à un évent ;
- la figure 6 est une représentation partielle d'une installation conforme à l'invention, la ligne tertiaire comprenant, en aval du point de connexion de la ligne d'analyse, un déverseur, la ligne d'analyse étant elle même pourvue d'un organe de création d'une perte de charge.

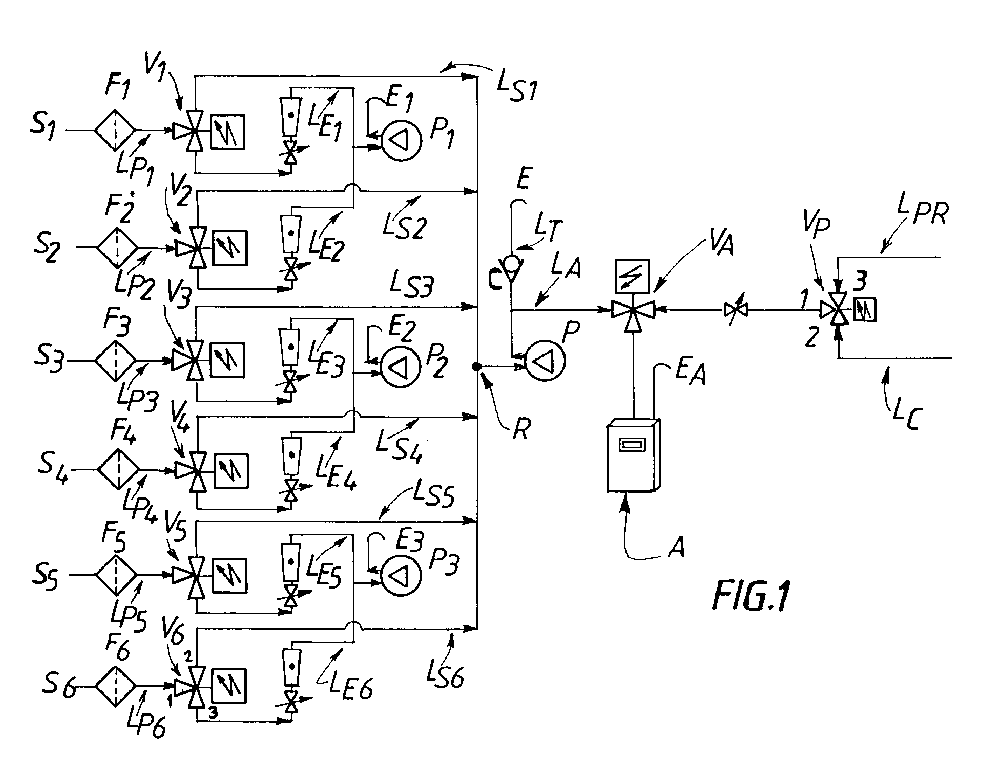

La figure 1 est une représentation schématique d'une installation conforme à l'invention, comportant six lignes de prélèvement Lpᵢ, chacune en provenance d'une source de gaz initiale Sᵢ, chaque ligne de prélèvement se connectant sur un organe directeur de flux Vᵢ, ici dans chaque cas, une électrovanne trois voies, en passant par un filtre Fᵢ.

Les sources initiales peuvent être par exemple constituées par des échantillons de gaz pris sur un réseau de distribution (application typique du contrôle d'impuretés avant autorisation d'introduction dans un procédé), ou encore par exemple être constituées par des échantillons de gaz pris en différents points d'une enceinte mettant en oeuvre une atmosphère gazeuse (four, tunnel, boite de stockage...).

Les sources initiales à analyser sont alors selon les cas à une pression supérieure à la pression atmosphérique (c'est le cas des réseaux de distribution où elle peut atteindre plusieurs bars, voire dizaines de bars), ou bien à une pression sensiblement égale à la pression atmosphérique ou inférieure à la pression atmosphérique. On sait par exemple que dans beaucoup d'enceintes mettant en oeuvre des gaz industriels comme des fours de traitement thermique ou des tunnels de traitement de produits alimentaires, la pression à l'intérieur de telles enceintes « ouvertes » est égale à la pression atmosphérique à quelques millibars près (P = PA + (1 à 50 mbars)).

Conformément à l'invention, sont connectées à chaque électrovanne trois voies Vᵢ, une ligne secondaire Lsᵢ (qui rejoint un point de rassemblement R), et une ligne d'évacuation L_{Ei}, qui rejoint pour le mode de réalisation représenté un évent Eᵢ, au travers d'un organe de pompage Pᵢ.

Comme on l'aura noté, pour le mode de réalisation représenté, on a regroupé deux lignes d'évacuation sur un même évent, par exemple les lignes d'évacuation 1 et 2 au niveau du même évent E₁, l'installation à six lignes représentées ne comportant alors que trois évents d'évacuation E₁, E₂ et E₃.

En poursuivant la description de l'installation de la figure 1 vers l'aval des lignes, on constate qu'une ligne tertiaire L_{T}, en provenance du point de rassemblement R, est apte à évacuer du gaz vers un évent E, en passant par un organe de pompage P et un clapet anti-retour C.

Sur cette ligne tertiaire, se pique une ligne d'analyse L_{A}, apte à amener du gaz à analyser vers un analyseur A, en passant par une électrovanne trois voies V_{A}.

L'analyseur A est également muni d'une sortie de gaz apte à évacuer le gaz reçu vers un évent E_{A}.

On notera par ailleurs que l'installation se complète par une électrovanne trois voies V_{P}, la combinaison des électrovannes V_{A} et V_{P} permettant selon les cas de diriger vers l'analyseur A du gaz à analyser (le long de la ligne L_{A}), du gaz de calibration (en provenance de la ligne L_{C}), ou encore un gaz de purge (en provenance de la ligne L_{PR}).

On a numéroté par les chiffres 1, 2 et 3 les trois voies de l'électrovanne V₆, afin d'en décrire plus confortablement le fonctionnement, et de façon générale le fonctionnement de l'installation de la figure 1.

Lorsque la bobine de l'électrovanne V₆ est excitée, le passage 1-2 est mis en oeuvre, c'est-à-dire que le gaz en provenance de la source S₆ est envoyé vers le point de rassemblement R. En revanche, quand la bobine de l'électrovanne V₆ n'est pas excitée, le passage 1-3 est alors mis en oeuvre, pour envoyer le gaz en provenance de la source S₆ vers l'évacuation E₃ le long de la ligne d'évacuation L_{E6}.

On conçoit alors que l'installation d'analyse en multiplexage de la figure 1 permet le prélèvement d'échantillons gazeux en six points différents et leur distribution vers l'analyseur, la conception de l'installation permettant, à l'aide d'un système de contrôle/commande non représenté sur la figure (tel un automate), un prélèvement continu des différents points analysés, en dirigeant vers l'analyseur A, au travers du point de rassemblement R, une source de gaz donnée, alors que tous les autres échantillons de gaz en provenance des sources Sᵢ sont dirigés vers les évacuations Eᵢ, puis ainsi de suite, la précédente source qui a fait l'objet d'une analyse est alors dirigée vers son évacuation alors que l'électrovanne d'une autre source est maintenant activée pour procéder à son analyse, laissant les autres intactes, c'est-à-dire dirigées vers les évacuations.

Une telle installation permet alors un prélèvement en continu au niveau des six sources de gaz initiales, minimisant le temps de réponse de l'analyseur, et assurant au niveau de cet analyseur des échantillons de gaz représentatifs de l'atmosphère au niveau des sources Sᵢ, en particulier dans le cas où l'analyseur est pour une raison ou pour une autre très éloigné des points de prélèvement.

On constate également qu'une telle installation permet de passer d'une source de gaz initiale à une autre, dans n'importe quel ordre, et la durée de consultation de chaque point de mesure est parfaitement configurable, à l'aide d'un système de commande déjà évoqué plus haut, permettant par exemple de suivre plus précisément certains points de mesure, lorsqu'ils sont par exemple plus sensibles.

De même, afin de répondre aux attentes de certains utilisateur souhaitant visualiser, voire archiver certaines données (par exemple l'évolution dans le temps de la teneur en un élément donné de l'atmosphère de leur enceinte de traitement), il est aisé d'utiliser l'installation selon l'invention en récupérant à des fins d'affichage ou de stockage des signaux provenant du ou des analyseurs de l'installation. Ces signaux sont également exploitables pour déclencher le cas échéant des alarmes selon un ou plusieurs niveaux détectés, ou encore pour effectuer sur leur base, à l'aide de l'automate, une régulation de la teneur d'un ou plusieurs éléments gazeux.

Considérons ici par exemple le cas où les sources de gaz initiales Si sont des points de prélèvement sur une ou plusieurs enceintes de mise en oeuvre de gaz industriels, par exemple des fours de traitement thermique sous atmosphère contrôlée, les gaz dans ces enceintes étant sensiblement à la pression atmosphérique.

Considérons alors par ailleurs également l'exemple du cas où l'analyseur A est muni de son propre organe de pompage, intrinsèque à l'analyseur.

On voit alors que quelle que soit la source de gaz initiale considérée (c'est-à-dire le point de prélèvement de gaz dans la ou les enceintes), le gaz prélevé arrive au point de rassemblement R et ensuite à la ligne tertiaire L_{T} à une pression sensiblement égale à la pression atmosphérique, l'analyseur A prélevant via son organe de pompage propre, uniquement le débit de gaz dont il a besoin pour fonctionner, via le piquage de la ligne L_{A} sur la ligne tertiaire, le reste du gaz arrivant au point de prélèvement R et qui n'est pas dirigé vers l'analyseur étant évacué le long de la ligne tertiaire vers l'évent E.

Les pompes P1, P2, P3 et P sont alors dimensionnées suivant la distance entre la baie d'analyse et les points de prélèvement, la pompe P étant notamment dimensionnée afin que le débit parvenant au point de rassemblement R et donc à la ligne tertiaire L_{T} soit supérieur au débit dont a besoin (et que va pomper) l'analyseur A pour fonctionner.

On constate aisément à la lecture de cette figure 1 que la combinaison des électrovannes V_{A} et V_{P} permet, par exemple durant des moments de pause ou encore de nuit lorsque l'installation est en veille, de diriger vers l'analyseur A un gaz de purge en provenance de la ligne L_{PR} ou encore un gaz de calibration en provenance de la ligne L_{_{C}}, l'ensemble des lignes de prélèvement L_{Pi} étant alors dans une telle configuration, soit carrément stoppé, soit balayé mais mis en communication avec ses évents Eᵢ associés.

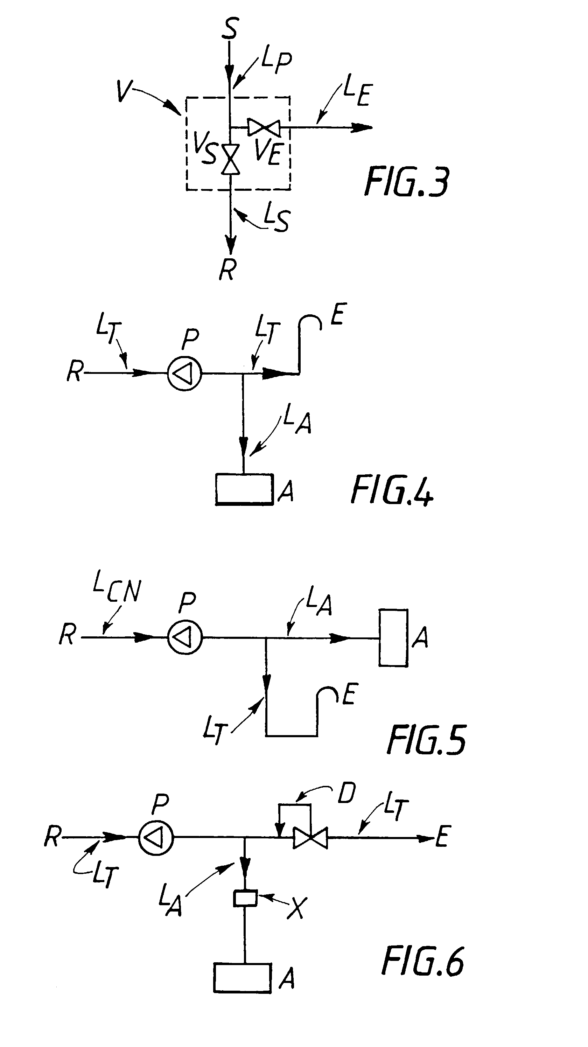

Si chaque organe directeur de la figure 1, et en particulier les vannes Vᵢ de chaque ligne de prélèvement, est constitué par une électrovanne trois voies, on peut bien entendu concevoir, sans sortir du cadre de la présente invention, d'autres types d'organes directeurs, tels qu'illustrés par exemple par le mode de réalisation de la figure 3, où ici l'organe directeur V est constitué par le point de connexion des ligne de prélèvement L_{P}, en provenance de la source S, de la ligne secondaire L_{S} se dirigeant vers le point de rassemblement R, et la ligne d'évacuation L_{E}, chacune des lignes L_{E} et L_{S} étant munie en aval du point, d'une vanne deux voies (respectivement V_{S} et V_{E}) permettant d'autoriser ou d'interdire le passage du gaz dans chaque ligne, permettant donc bien de diriger le gaz en provenance de la source de gaz initiale S tour à tour, selon les besoins, vers le point de rassemblement R ou bien vers l'évacuation.

Comme il apparaîtra clairement à l'homme du métier, si l'installation illustrée figure 1 représente un mode de réalisation où la ligne d'analyse L_{A} se pique sur la ligne tertiaire L_{T}, d'autres configurations sont envisageables, sans sortir du cadre de la présente invention, et doivent être considérées comme entrant sous la formulation générale utilisée plus haut dans cette description selon laquelle l'installation comporte "au moins une ligne d'analyse reliée en sa partie amont à la ligne tertiaire et en sa partie aval ....". Ainsi la représentation partielle de la figure 4 reprend la configuration de connexion de la figure 1, alors que la figure 5 illustre une variante que l'on décrirait plutôt par le fait que la ligne tertiaire se pique sur la ligne d'analyse L_{A}, les deux formulations étant interchangeables :
- les deux lignes (d'analyse et tertiaire) sont dans tous les cas "reliées" (piquage) au niveau d'un point, et
- on peut considérer que dans le cas de la figure 5 également, la ligne tertiaire est « reliée en sa partie amont au point de rassemblement, même si c'est au travers d'une section de ligne « de connexion » symbolisée sur la figure par L_{CN}.

On comprend alors que l'appellation n'a qu'une importance secondaire et qu'il faille avant toute chose s'attacher à retrouver la finalité réelle de chaque ligne.

On notera cependant que la configuration de la figure 1 et de la figure 4 est avantageuse par le fait que le gaz prélevé par la ligne d'analyse L_{A} sur la ligne tertiaire L_{T} est plus sûrement à la pression atmosphérique puisque se dirigeant vers l'évent E.

En revenant maintenant à la description détaillée de la figure 1, si l'on a envisagé précédemment un exemple d'application où l'analyseur A était muni de son propre organe de pompage, on peut également, bien entendu, envisager un analyseur A ne possédant pas son propre organe de pompage, il est alors avantageux d'adopter selon l'invention la configuration schématisée dans le cadre de la figure 6, où la ligne tertiaire L_{T}, en aval du point de connexion de la ligne d'analyse L_{A} sur la ligne tertiaire, comporte un déverseur D, alors que la ligne d'analyse L_{A} est elle même munie d'un organe X de création d'une perte de charge.

Cet organe de création d'une perte de charge pourra être constitué de façon très variée d'un organe tel qu'une vanne de laminage, ou encore une vanne d'arrêt, un limiteur de débit, un orifice calibré, ou encore de façon très générale, ce moyen de création d'une perte de charge peut également être obtenu par la configuration particulière de tuyauterie utilisée à cet endroit de la ligne.

On conçoit alors que l'ensemble constitué du déverseur dans la ligne tertiaire (un déverseur étant considéré comme un régulateur de pression amont) et de l'organe de création d'une perte de charge dans la ligne d'analyse permettra d'assurer un débit constant dans la ligne d'analyse et donc parvenant à l'analyseur, ceci quel que soit le point de prélèvement considéré, et la pression du gaz au niveau de la ligne de prélèvement considérée, par le fait que la pression dans la ligne tertiaire et dans la ligne d'analyse est parfaitement fixée, quelles que soient les variations pouvant intervenir en amont.

La même remarque effectuée précédemment concernant le fait de considérer un piquage de la ligne d'analyse sur la ligne tertiaire ou de la ligne tertiaire sur la ligne d'analyse pourrait être reprise ici.

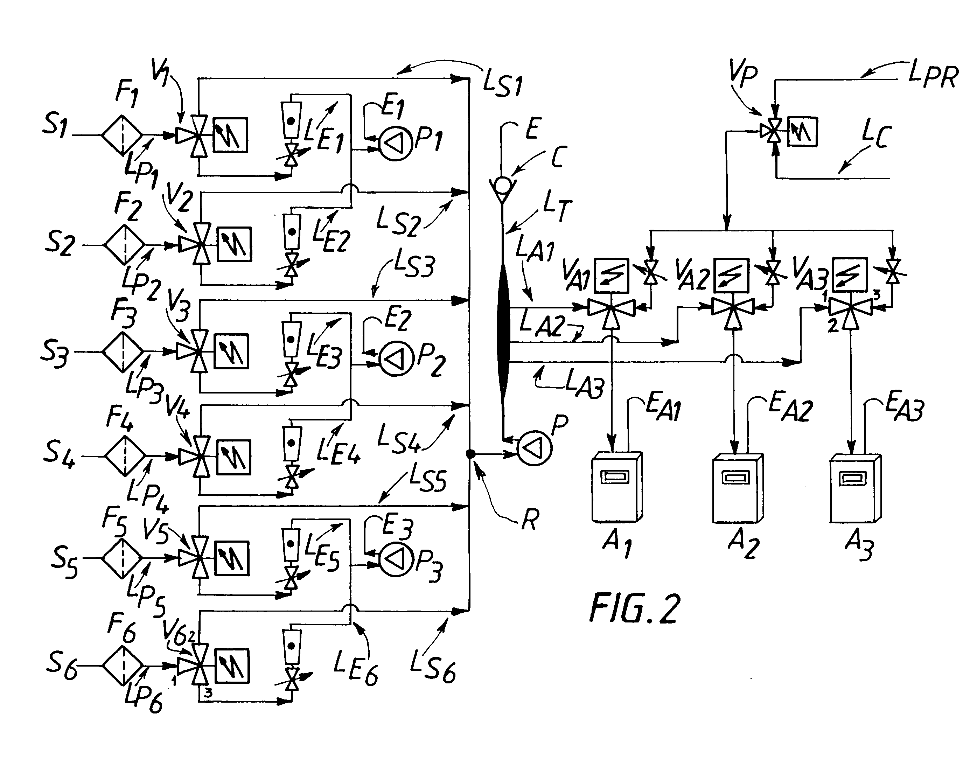

Si la figure 1 illustre une installation d'analyse conforme à l'invention comportant un seul analyseur A, la figure 2 illustre pour sa part une installation d'analyse conforme à l'invention comportant trois analyseurs A₁, A₂ et A₃.

L'installation de la figure 2, en amont du point de rassemblement R, est alors inchangée par rapport à l'installation de la figure 1, on se contentera alors dans ce qui suit de décrire l'aval du point de rassemblement R.

L'installation comporte ici une ligne d'analyse par analyseur (respectivement L_{A1}, L_{A2} et L_{A3}), chaque ligne d'analyse étant connectée sur la ligne tertiaire L_{T} au niveau d'une nourrice, représentée sur la ligne tertiaire par une forme sombre oblongue.

Chaque ligne d'analyse comporte par ailleurs un organe directeur constitué ici d'une électrovanne trois voies (V_{A1}, V_{A2} et V_{A3}).

Comme précédemment, chaque analyseur est ici encore muni d'une évacuation de gaz permettant l'évacuation du gaz reçu vers un évent, respectivement E_{A1}, E_{A2} et E_{A3}.

On notera par ailleurs la présence sur l'installation d'une électrovanne V_{P}, reliée à chaque électrovanne VAᵢ, et apte à diriger sur chaque électrovanne V_{Ai} et donc sur chaque analyseur, un gaz de purge (en provenance de la ligne L_{PR}) ou un gaz de calibration (en provenance de la ligne de calibration L_{C}).

On voit donc qu'une telle installation permet l'analyse d'un ou plusieurs composants pour chaque point de prélèvement, tout en maintenant tous les avantages déjà signalés dans le cadre de la figure 1 liés au prélèvement en continu des différents points analysés en amont du point de rassemblement R.

Bien entendu les variantes déjà décrites dans le cadre des figures 3 à 6 pourraient également être adoptées dans le cadre de cette installation de la figure 2 (organe directeur constitué d'un T et de deux vannes deux voies, ou encore analyseurs non munis de leur propre organe de pompage alimentés au travers d'un organe de création d'une perte de charge).

Ici encore on a numéroté 1, 2 et 3, les trois voies de l'électrovanne V_{A3} afin d'expliciter plus clairement le fonctionnement de cette électrovanne et de façon générale de l'installation.

Quand la bobine de l'électrovanne V_{A3} est excitée, le passage 1-2 est mis en oeuvre alors que lorsque la bobine de l'électrovanne V_{A3} n'est pas excitée, c'est le passage 1-3 qui est mis en oeuvre.

Considérons maintenant l'exemple d'un cas où le gaz en provenance de la source S₆ est dirigé vers le point de rassemblement R, tous les autres prélèvements en provenance des autres sources S₁ à S₅ étant dirigés vers leur évent respectif E1 à E3.

Si le gaz en provenance de S₆ doit être analysé par exemple par l'analyseur A₃, la bobine de l'électrovanne V_{A3} est alors excitée (par un système de commande annexe que l'on ne décrira pas ici, systèmes de commande déjà évoqués dont l'homme du métier de l'analyse et du contrôle de procédés est couramment familier), permettant le transit du gaz selon le passage 1-2 alors que les autres électrovannes V_{A1} et V_{A2} sont non-excitées, permettant le balayage des analyseurs A₁ et A₂ par du gaz de purge en provenance de l'ensemble V_{P}/L_{PR}.

Bien entendu, si un échantillon de gaz en provenance de la source S₆ devait ensuite également être analysé par un autre des analyseurs A₁ ou A₂, il serait alors parfaitement possible de ne plus exciter la bobine de l'électrovanne V_{A3}, pour permettre la purge de l'analyseur A₃ par du gaz de purge, et exciter la bobine par exemple de l'électrovanne V_{A2} pour permettre le passage de l'échantillon de gaz en provenance de la source S₆ vers l'analyseur A₂, l'électrovanne V_{A1} demeurant alors non excitée, et l'analyseur A₁ purgé.

## Revendications

1. Installation d'analyse de la teneur en au moins un élément, d'au moins deux sources (S₁,S₂,..) de gaz initiales, sur un mode d'échantillonnage, à l'aide d'au moins un analyseur (A₁, A₂..) apte à analyser ledit élément, comportant :
- au moins deux sources (S₁,S₂,..) de gaz initiales à analyser ;
- au moins deux lignes de prélèvement (L_{P1},L_{P2},..), chaque ligne étant reliée en sa partie amont à une desdites sources de gaz initiales, et en sa partie aval, à un organe (V₁,V₂,..) respectif directeur de flux ;
- au moins deux lignes d'évacuation (L_{E1},L_{E2},..), chaque ligne d'évacuation étant reliée en sa partie amont à un desdits organes directeurs et en sa partie aval à une évacuation ou à un point de stockage (E₁,E₂,..);
- au moins deux lignes secondaires (L_{S1},L_{S2},..), chaque ligne secondaire étant reliée en sa partie amont à un desdits organes directeurs et, en sa partie aval, à un point de rassemblement (R), chaque organe directeur étant apte à diriger un échantillon de la source de gaz initiale qui lui est associée vers sa ligne d'évacuation respective ou vers le point de rassemblement via sa ligne secondaire respective ;
- une ligne tertiaire (L_{T}), reliée en sa partie amont au point de rassemblement et en sa partie aval à une évacuation ou à un point de stockage (E);
- au moins une ligne d'analyse (L_{Ai}), reliée en sa partie amont à la ligne tertiaire et en sa partie aval à au moins un desdits au moins un analyseur.

2. Installation selon la revendication 1, caractérisée en ce que la ligne tertiaire comporte un organe de pompage (P), situé entre le point de rassemblement et le point de connexion entre la ligne tertiaire et ladite au moins une ligne d'analyse.

3. Installation selon la revendication 1 ou 2, caractérisée en ce que la ligne tertiaire comporte un clapet anti-retour, en aval du point de connexion de ladite au moins une ligne d'analyse sur la ligne tertiaire.

4. Installation selon l'une quelconque des revendications 1 à 3, caractérisée en ce que la ligne tertiaire comporte, en aval du point de connexion de ladite au moins une ligne d'analyse sur la ligne tertiaire, un déverseur (D), et en ce que au moins une des lignes d'analyse est munie d'un organe (X) de création d'une perte de charge.

5. Installation selon l'une quelconque des revendications 1 à 4, caractérisée en ce que la ou au moins une des lignes d'évacuation est munie d'un organe de pompage (Pᵢ).

6. Installation selon l'une quelconque des revendications 1 à 5, caractérisée en ce que la où chaque ligne d'analyse est munie d'un organe directeur (V_{Ai}) permettant selon les cas de diriger vers l'analyseur du gaz à analyser, un gaz de calibration, ou un gaz de purge.

7. Installation selon l'une quelconque des revendications 1 à 6, caractérisée en ce que le ou lesdits organes directeurs sont constitués par une électrovanne trois voies.

8. Installation selon l'une quelconque des revendications 1 à 6, caractérisée en ce que le ou lesdits organes directeurs des lignes de prélèvement sont constitués par l'ensemble formé par le point de connexion des lignes de prélèvement, d'évacuation, et secondaire associées, chaque ligne secondaire et d'évacuation étant munie, en aval de ce point, d'un organe apte à permettre ou interrompre le passage de gaz par la ligne considérée.

9. Installation selon l'une quelconque des revendications 1 à 8, caractérisée en ce que lesdites sources initiales sont à une pression supérieure à la pression atmosphérique.

10. Installation selon l'une quelconque des revendications 1 à 8, caractérisée en ce que lesdites sources initiales sont à une pression sensiblement égale à la pression atmosphérique ou inférieure à la pression atmosphérique.

11. Installation selon la revendication 9, caractérisée en ce que lesdites sources de gaz initiales sont des prises d'analyse sur un réseau de distribution de gaz.

12. Installation selon la revendication 10, caractérisée en ce que lesdites sources initiales sont des prises d'analyse en différents points d'une enceinte mettant en oeuvre une atmosphère gazeuse.

13. Installation selon l'une des revendications 1 à 12, caractérisée en ce qu'elle comporte plusieurs analyseurs, et en ce que la ou les lignes d'analyse sont connectées sur la ligne tertiaire au niveau d'une nourrice.

14. Installation selon la revendication 13, caractérisée en ce qu'elle comporte une seule ligne d'analyse connectant la ligne tertiaire et les analyseurs disposés en série.

15. Installation selon la revendication 13, caractérisée en ce qu'elle comporte une ligne d'analyse par analyseur.
